# EUROPEAN PATENT APPLICATION

(11) **EP 1 484 025 A1**
(43) Date of publication of application: **08.12.2004**
(21) Application number: 04021722.6
(22) Date of filing: 05.03.1999
(51) Int. Cl.: A61B 18/14, A61B 17/22

(54) **Catheter assembly with electrode**

(30) Priority: 05.03.1998 US 35738; 05.03.1998 US 35737
(62) Divisional of application: 99911188.3
(71) Applicant: Boston Scientific Limited, St. Michael, Barbados (BB)
(72) Inventor: The designation of the inventor has not yet been filed
(74) Representative: VOSSIUS & PARTNER

(57) **Abstract**

The invention relates to a catheter assembly comprising an elongate shaft having a proximal end and a distal end and including a conductor therein, an insulator disposed around the conductor, a conductive loop attached to the conductor, disposed at the distal end of the shaft, the conductive loop having a distal end, an electrode disposed at the distal end of the loop. The loop can act as a shock absorber to dampen the change in force on the electrode incident to a heart beat.

## Description

### Field of the Invention

The present invention relates generally to medical devices for forming holes in heart chamber interior walls in percutaneous myocardial revascularization (PMR) procedures. More specifically, the present invention relates to intravascular PMR devices having expandable distal loops deployable within heart chambers.

### Background of the Invention

A number of techniques are available for treating cardiovascular disease such as cardiovascular by-pass surgery, coronary angioplasty, laser angioplasty and atherectomy. These techniques are generally applied to by-pass or open lesions in coronary vessels to restore and increase blood flow to the heart muscle. In some patients, the number of lesions are so great, or the location so remote in the patient vasculature that restoring blood flow to the heart muscle is difficult. Percutaneous myocardial revascularization (PMR) has been developed as an alternative to these techniques which are directed at by-passing or removing lesions.

Heart muscle may be classified as healthy, hibernating and "dead". Dead tissue is not dead but is scarred, not contracting, and no longer capable of contracting even if it were supplied adequately with blood. Hibernating tissue is not contracting muscle tissue but is capable of contracting, should it be adequately re-supplied with blood. PMR is performed by boring channels directly into the myocardium of the heart.

PMR was inspired in part by observations that reptilian hearts muscle is supplied primarily by blood perfusing directly from within heart chambers to the heart muscle. This contrasts with the human heart, which is supplied by coronary vessels receiving blood from the aorta. Positive results have been demonstrated in some human patients receiving PMR treatments. These results are believed to be caused in part by blood flowing from within a heart chamber through patent channels formed by PMR to the myocardial tissue. Suitable PMR holes have been burned by laser, cut by mechanical means, and burned by radio frequency current devices. Increased blood flow to the myocardium is also believed to be caused in part by the healing response to wound formation. Specifically, the formation of new blood vessels is believed to occur in response to the newly created wound.

What would be desirable is a device capable of forming multiple holes in the wall of a heart chamber but requiring minimal manipulation while positioned within the chamber. What would be desirable is a device capable of forming multiple holes in a short time period in the myocardium.

### Summary of the Invention

The present invention provides devices and methods for forming a plurality of holes in a heart chamber wall as part of a percutaneous myocardial revascularization (PMR) treatment. Devices according to the present invention have a basket, termed a PMR basket, formed of a plurality of arcuately biased flexible arms. The arms can assume an outwardly bowed configuration once freed of the confines of a shaft lumen. The fully deployed PMR basket arms can expand until the arms engage the walls of the heart chamber. Some devices provide for multiple, simultaneous myocardial hole formation. In such devices, cutting means such as RF electrodes are carried on the arms and disposed outwardly to engage the heart chamber walls. The term "cutting" as used herein, means penetration, including the formation of holes by burning and by other means. One device utilizes a PMR basket to anchor or stabilize a steerable PMR cutting probe within the heart chamber.

The present invention provides devices and methods for forming numerous holes in a heart chamber wall within a short time period. This reduces the amount of time the heart chamber is invaded by the foreign device. Some devices and methods according to the present invention allow for the formation of many holes while requiring minimal maneuvering once the devices are advanced into the heart chamber.

One group of PMR basket devices includes an elongate outer tubular shaft having a lumen, a proximal end and a distal end. A plurality of elongate flexible arms are secured together at their proximal and distal ends, the arms being biased so as to bow outward relative to an axis through the secured proximal and distal ends. The arms carry a plurality of cutting means disposed in an outward direction toward the chamber wall. One device arms have lumens therethrough and outwardly oriented apertures. This device includes electrical supply wires running though the arms lumens and electrode wires extending from the supply wires and through the arm apertures. This device can include arcuately biased electrode wires that can be formed of a shape memory material. The biased arms can extend radially outward away from the arm when unconstrained, especially when heated by body fluids. One device includes a single supply wire slidably disposed within the arm lumen and electrically connected to each of the electrode wires which are slidably disposed within an arm aperture. The electrode wires can be advanced away from the apertures by advancing the supply wire within the arm. A variation of this embodiment utilizes cutting probes having sharpened free ends capable of piercing the heart chamber wall and forming holes within the myocardium.

In another embodiment of the PMR basket, the basket includes a plurality of electrode groupings. The electrodes can also have a loop shape, which can be, for example, semi-circular. Both the electrode grouping and loop shaped electrodes can be used to form craters in the myocardium of a patient's heart rather than channels. Craters can be considered wounds which have a width greater than their depth, whereas channels can be considered to a have a depth greater than their width. A hole is the resulting space after volumetric removal of material has been made from the patient's heart wall. A hole can be either a crater or a channel.

It is believed that forming craters, in some instances, provide better therapeutic value than forming channels in the myocardium as their formation can be better controlled to reduce the likelihood of heart wall perforation.

Another embodiment of the device includes an electrode which in most embodiments has a width greater than its depth such that it can be used to form craters in the myocardium of a patient's heart rather than channels. Craters are wounds in the myocardium of a patient's heart which have a width greater than their depth whereas channels can be considered to have a depth greater than their width. Holes in the myocardium are volumetric removals of material. The embodiments also include a loop to limit the penetration of the electrode.

In one embodiment, a catheter assembly is provided including an elongate shaft having a proximal and a distal end and a conductor extending through the shaft. An insulator is disposed around the conductor. A conductive loop is disposed at the distal end of the shaft. The conductive loop in turn has an electrode disposed at its distal end.

The catheter shaft can include a proximal portion and a more flexible distal portion. The proximal and distal portions can be stainless steel and Nitinol hypotubes respectively.

The loop is preferably formed from Nitinol which can be heat set to expand on introduction of the loop into a chamber of a patient's heart. The loop is preferably insulated with a material such as PTFE which can withstand high temperatures. The distal end of the loop, however, can act as an electrode if uninsulated.

The electrode can be positioned at the distal end of the loop. Preferably the electrode is substantially radiopaque such that it can readily be viewed by fluoroscopy.

In yet another embodiment, the loop is disposed proximate and proximally of the distal end of the shaft. An electrode is disposed at the distal end of the shaft. The shaft defines an elongate lumen. The lumen continues through the electrode such that contrast medium, growth factors or other drugs can be delivered to the wound created by the PMR procedure. The loop, if insulated, can act as a stop to limit the penetration of the electrode.

In yet another embodiment, the loop is retractable within the catheter shaft. In this embodiment, an elongate reciprocating shaft is disposed within a lumen defined by the catheter shaft. The reciprocating shaft is connected to at least one end of the loop, the reciprocating shaft is moveable between a first position and a second position such that in the second position, the loop has a greater transverse dimension than in the first position. In the first position, the reciprocating shaft is used to withdraw the loop into the catheter shaft lumen to reduce the transverse dimension of the loop for delivery and withdrawal from the heart.

In the method in accordance with the present invention, a catheter assembly is provided including an elongate shaft having a proximal end and a distal end. A transversely expandable conductive loop is disposed at the distal end of the shaft. An electrode is disposed on the loop. The loop is advanced to the myocardium of the patient's heart where the transverse dimension of the loop is allowed to expand as the loop enters a chamber of the patient's heart. The electrode is advanced to the endocardium and energized to form a crater in the myocardium. The electrode can be repeatedly advanced to the myocardium to form a plurality of craters. The electrode is preferably energized with radiofrequency energy to create an arc which ablates or removes tissue.

### Brief Description of the Drawings

Figure 1 is a perspective, cutaway view of a human heart having a PMR basket device inserted over the aorta and into the left ventricle;
Figure 2 is a fragmentary, perspective view of the PMR basket device of Figure 1, having cutting probes and flexible arms in an expanded state;
Figure 3 is an expanded, cutaway view of detail area 3 of Figure 2, having a PMR basket cutting probe slidably disposed within an arm;
Figure 4 is fragmentary, side view of a PMR basket cutting probe wherein the probe is an electrode protruding from an insulated arm;
Figure 5 is fragmentary, side view of a PMR basket cutting probe wherein the probe is a needle having a lumen and a sharp point;
Figure 6 is fragmentary, side view of a PMR basket arm wherein the cutting means including a water jet orifice supplied by the flexible arm lumen;
Figure 7 is fragmentary, side view of a PMR basket arm having an insulated, electrical cutting probe;
Figure 8 is a fragmentary, perspective view of a PMR basket anchor and steerable PMR cutting probe disposed within the basket;
Figure 9 is a fragmentary, perspective view of a PMR brush device having a plurality of arcuately biased electrical cutting probes, the device being in an expanded state;
Figure 10 is a fragmentary, side, cutaway view of the PMR brush device of Figure 9 in a retracted, compact state;
Figure 11 is the perspective view of a PMR basket having a plurality of electrode groupings disposed thereon;
Figure 12 is a fragmentary view of an electrode grouping from Figure 11;
Figure 13 is a perspective view a PMR basket having a plurality of loop electrodes disposed thereon;
Figure 14 is a fragmentary view of a loop electrode from Figure 13;
Figure 15 is a longitudinal, cross-sectional view of a PMR catheter in accordance with the present invention;
Figure 16 is a perspective view of an alternate embodiment of the distal end of the PMR catheter;
Figure 17 is a perspective view of yet another alternate embodiment of the distal end of the PMR catheter;
Figure 18 is a perspective view of yet another alternate embodiment of the distal end of the PMR catheter;
Figure 19 is a perspective view of yet another alternate embodiment of the distal end of the PMR catheter; and
Figure 20 is a view of the distal end of the PMR catheter of Figure 19 shown in a deployed position.

### Detailed Description of the Preferred Embodiments

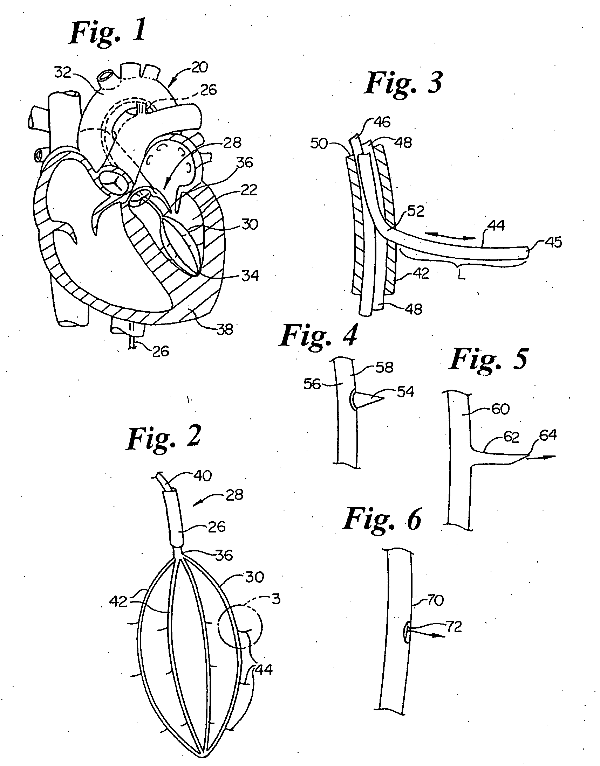

Referring to Figure 1, a human heart 22, including an apex 38, a left ventricle 22 and an aorta 32, is illustrated having a PMR basket device 28 disposed within ventricle 22. PMR basket device 28 includes an elongate outer shaft 26 lying within aorta 32 and a basket 30 disposed within left ventricle 22. PMR basket 30 includes a distal portion 34 and a proximal portion 36, with distal portion 34 disposed near apex 38.

Referring now to Figure 2, PMR device 28 is illustrated in more detail, having an inner shaft 40 slidably disposed within outer shaft 26. PMR basket 30 includes a plurality of flexible arms 42 secured together at distal portion 34 and proximal portion 36. In one embodiment, PMR device 28 is constructed in a similar manner to the electrophysiological mapping device disclosed in U.S. Patent No. 5,628,313 (Webster, Jr.), herein incorporated by reference. In particular, the construction of arms 42, shafts 26 and 40, and their interconnections can be similar. Flexible arms 42 include several cutting probes 44 depicted generally in Figure 2. In a preferred embodiment, cutting probes 44 have a length "L" extending from flexible arms 42 that decreases with increasing distal distance along the device. Thus, in a preferred embodiment, the length of cutting probes 44 is least near distal portion 34. A shorter cutting probe length is desirable as the heart wall is generally thinner near the apex. As can be appreciated, the basket might also be used as a probe to map conductive versus non-conductive myocardial tissue.

As can be seen from inspection of Figure 2, proximally retracting inner shaft 40 within outer shaft 26 will result in arms 42 being proximally withdrawn into outer shaft 26, thereby collapsing basket 30. Conversely, distally advancing inner shaft 40 results in arms 42 being distally advanced as well, freeing arms 42 from the constraint of shaft 26. Arms 42 have an arcuate outward bias as illustrated in Figure 2. The arcuate bias allows arms 42 to expand and form basket 30 once freed from the confines of outer shaft 26. Arms 42 are preferably formed of a shape memory material such as Nitinol, which can assist in the formation of expanded basket 30, as arms 42 re-attain an outwardly bowed, arcuate shape. As can be seen from inspection of Figure 1, basket 30 can be further expanded by distally forcing inner shaft 40 against basket proximal portion 36, thereby pushing basket distal portion 34 against the ventricle wall and forcing arms 42 even further apart. The longitudinally directed force of inner shaft 40 is thus partially transmitted into radially directed forces over cutting probes 44. In embodiments where the cutting probes are sharpened needles, the radial force can operate to force the needles into the heart chamber wall. In embodiments where cutting probes are electrical, the radial force can operate to bring the cutting probes into sufficiently close proximity to the heart chamber wall to allow burning holes into the heart chamber wall and myocardium.

In one embodiment, cutting probes 44 are formed of Nitinol or other shape memory material and have a bias or preform of their own. One bias is an arcuate bias as illustrated in Figure 2. Cutting probes in this embodiment lie flat against arms 42 while the arms are retracted within outer shaft 26 and are allowed to assume a remembered; arcuate shape upon release from the constraint of outer shaft 26 and exposure to warm body fluids. Once in the arcuate position, cutting probes 44 are better able to contact the ventricle walls. In another embodiment, not requiring illustration, the cutting probes are formed of Nitinol and wound into a tight spiral which extends and lengthens the spiral into a less tightly wound spiral or straighter wire upon assuming the remember shape. The spiral wound embodiment also allows the cutting arms to extend to the ventricle walls.

Referring now to Figure 3, an enlargement of cutting probe 44 illustrates an extensible length "L", extending radially from the generally longitudinal orientation of arm 42. Cutting probe 44 terminates in a cutting tip 45. In a preferred embodiment, cutting probe 44 is an electrode and cutting tip 45 cuts into the ventricle wall due to the application of an electrical signal, preferably a radio frequency (RF) current, to the ventricle walls. A lumen 50 in arm 42 contains a first supply wire 46 which is bonded to, and electrically connected to, cutting probe 44. In the embodiment illustrated, a second supply wire 48 also extends through arm 42, for interconnection to a cutting probe. In one embodiment, more than one supply wire runs within arm lumen 50. In one lumen, a unique supply wire can exist for every electrode wire. In a preferred embodiment, a single supply wire runs within each arm and is electrically connected to multiple electrode wire cutting probes. Unlike electrical mapping, delivery of current for burning multiple myocardial holes does not absolutely require distinct multiple supply wires. In one embodiment, supply wire 46 slides longitudinally within arm 42 and cutting probe 44 slides radially through an orifice 52 in arm 42. Thus, longitudinally sliding the supply wire causes cutting probe 44 to slide radially toward the heart chamber wall, as indicated by the arrows in Figure 3. In a preferred embodiment, electrical insulation coats either the exterior of supply wire 46 or the interior of arm lumen 50, or both.

Referring now to Figure 4, another arm 58 and cutting means is illustrated in Figure 4 in a cutting electrode 54 having an electrically insulating coating 56 over arm 58, leaving only cutting electrode 54 exposed. The arm embodiment of Figure 4 allows use of smaller diameter arms as no lumen is required as the insulation is external to the arm. This can allow more arms to be fit within outer tube 26 when retracted. A greater number of arms in the basket allows the formation of a greater number of holes simultaneously formed. In use, after expanding the PMR basket, cutting electrode 54 is brought into close proximity to the heart chamber wall and sufficient electrical potential supplied to a supply wire to burn a hole in the heart chamber wall.

Referring now to Figure 5, another arm 60 is illustrated, having a cutting needle 62 terminating is a cutting tip 64. In one embodiment, needle 62 is formed of a solid material such as stainless steel. In another embodiment, needle 62 contains a lumen and cutting tip orifice such that a lumen through arm 60 can supply a high pressure fluid through cutting needle 64 and cutting tip 64. Needle 64 can be used in some methods to deliver angiogenic materials in conjunction with the hole formation by cutting tip 64. For example, angiogenic material can be delivered into a hole recently burned by RF current delivered through needle 62. Contrast media may also be delivered through needle 62.

Referring now to Figure 6, another arm 70 is illustrated, having a cutting orifice 72 used to deliver fluid under high pressure to form myocardial holes. Cutting orifice 72 supplies sufficiently high pressure fluid to form holes without requiring the use of electrodes or sharp needles. In use, arm 70 is brought into close contact with a heart chamber wall and high pressure fluid forced through the arm and other arms.

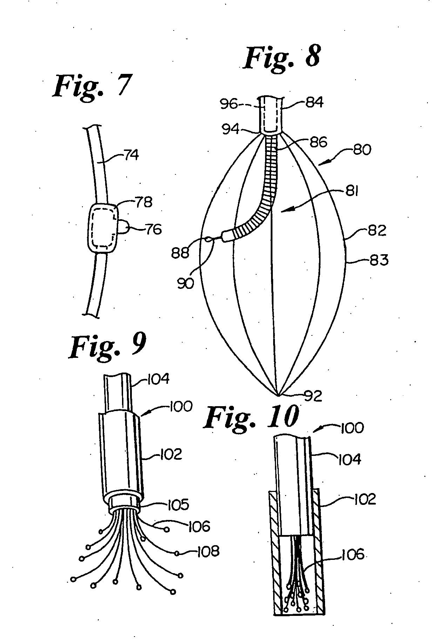

Referring now to Figure 7, yet another embodiment is illustrated in an arm 74 having a burning electrode 76 protruding from an insulated housing 78. Insulated housing 78 and electrode 76 allow a conventional electrophysiological mapping basket to be converted into a PMR cutting device. Insulating housing 78 can be formed of a material such as Teflon® or other thermoplastic such as PEBA. Burning electrode 76 has a rounded distal tip, to form a crater shaped burn in the heart chamber wall. Other cutting electrodes, in particular, longer and sharper tipped electrodes, can also be used with the embodiment of Figure 7.

Referring now to Figure 8, a PMR basket device 80 is illustrated. PMR device 80 includes a basket 82 formed of a plurality of arms 83 terminating in distal portion 92 and proximal portion 94. An outer shaft 84 is slidably disposed over an intermediate shaft 96 which can have an inner, steerable cutting probe 81 disposed within. In one embodiment, intermediate shaft 96 functions as a hub, having arms 83 secured thereto and steerable probe 81 slidably and rotatably received within. Steerable cutting probe 81 includes a shaft 86 preferably having a lumen therethrough. Basket 82 can be similar, in shape and arcuate bias of individual arms, to basket 30 in Figure 1, but having no means for cutting mounted over the length of the arms. Basket proximal portion 94 is affixed to the distal end of intermediate shaft 96. In one embodiment, as in shaft 96 in Figure 8, the intermediate shaft is tubular, having cutting probe shaft 86 slidably disposed within.

In another embodiment, the intermediate shaft and cutting probe shaft 86 are both slidably disposed, side-by-side, within outer shaft 84. Cutting probe shaft 86 is preferably arcuately biased and slidably disposed within outer shaft 84 such that the degree of arc exhibited by the probe can be controlled by longitudinally advancing or retracting the arcuately biased member within the constraints of outer shaft 84. Allowing more of cutting probe shaft 86 to extend distally from outer shaft 84 allows more the arcuate shape to be attained. In one embodiment, cutting probe shaft 86 is formed of a shape memory material such as Nitinol. In another embodiment, cutting probe shaft 86 is formed of a spiral wound material such as woven stainless steel braid in a polymer which is biased to have an unconstrained arcuate shape. A cutting wire 90 is preferably slidably disposed within shaft 86 and can terminate in a distal cutting tip 90. In another embodiment, an elastic fabric may be suspended around basket 82 to form a balloon-like enclosure. A plurality of apertures may be formed in the fabric such that cutting tip 88 can exit the basket to access the myocardium only through a hole in the fabric. The hole may be placed in a predetermined array to guide the arrangement of channels or craters to be formed during the PMR procedure.

In use, intermediate shaft 96, basket 82 and cutting probe 81 can be proximally retracted within outer shaft 84 in preparation for placement. The distal end of outer shaft 84 can be advanced over the aorta and into a heart chamber such as the left ventricle. Intermediate shaft 96 can be advanced distally into the left ventricle, forcing basket 82 out from within outer shaft 84. Cutting probe shaft 86 can also be advanced distally from outer shaft 84, either separately or together with basket 82. Basket 82, freed from the constraint of outer shaft 84, can assume the outwardly bowed arcuate shape imparted by arms 83. Further bowing can be achieved by distally pushing intermediate shaft 96, thereby forcing basket distal portion 92 against the ventricle wall near the apex. Basket 82 thus acts as an anchor, stabilizing the position of cutting probe 81 within the ventricle.

In one embodiment, the shape of cutting probe 81 can be controlled in part by imparting to cutting probe shaft 86 an arcuate bias arid controlling the length of shaft 86 that is allowed to extend from within outer shaft 84 and inner shaft 96. With the degree of arc thus controlled, cutting wire 90 can be advanced until contact with the heart chamber wall is effected. With cutting tip 88 in contact with the chamber wall, a suitable RF electrical current can be passed through cutting wire 90, thereby burning a hole in the heart chamber wall. Cutting probe 81 can be rotated, allowing a circle or arc of myocardial holes to be formed within the heart chamber. Adjusting the longitudinal position of cutting probe 81 allows other series of holes to be formed. In one embodiment (not requiring illustration), an additional tube is slidably disposed over cutting probe shaft 86, distally past intermediate shaft 94, and within basket 82, allowing control of the arc of cutting probe shaft 86 to be extended longitudinally. While anchoring basket 82 is preferably used in conjunction with electrical PMR cutting means, other cutting means, including sharp cutting tips, are used in other embodiments.

Referring now to Figure 9, a PMR brush device 100 is illustrated, including an inner shaft 104 having a distal end 105 and a plurality of arcuate electrode wires 106 secured to distal end 105. Inner shaft 104 is slidably disposed within an outer shaft 102. Electrode wires 106 can terminate in a distal cutting tips 108 which, in one embodiment, are formed of metallic balls of platinum or gold brazed to the distal ends of electrode wires 106. In one embodiment, electrode wires 106 are insulated except for cutting tip 108. Electrode wires 106 can be formed of a shape memory material such as Nitinol. The electrodes can be formed into an initial arcuate shape, with the shape being remembered after the electrodes are freed from constraint and warmed to body temperature. Referring now to Figure 10, inner shaft 104 is illustrated in a retracted position within outer shaft 102.

In use, PMR brush device 100 can be put into a retracted position as illustrated in Figure 10 and advanced over the aorta and into a heart chamber such as the left ventricle. Outer shaft 102 can be retracted, freeing electrode wires 106 of the restraint of shaft 102, allowing the wires to assume an arcuate shape such that cutting tips 108 can engage the ventricle wall. With cutting tips 108 in position, a suitable electrical source can be switched, causing cutting tips 108 to burn holes into the heart chamber wall. In one embodiment, electrode wires 106 are supplied by a common supply wire and all electrodes fired simultaneously.

PMR brush device 100 can be made to engage the ventricle walls at varying depths within the ventricle. For example, PMR device 100 can have outer shaft 102 only partially retracted, keeping arcuate electrode wires 106 partially straightened and grouped together for engaging the ventricle wall near the apex. After burning a series of holes, outer tube 102 can be retracted further, allowing arcuate electrode wires 106 to splay further apart, allowing a superior, wider portion of the left ventricle to be treated. In one embodiment, electrode wires 106 are preformed to have an extreme arcuate shape such that superior, wide regions of the left ventricle can be treated from a central position in the ventricle. In some methods, brush device 100 may be pulled via inner shaft 104 to more completely treat the right wall of the left ventricle and pushed to more completely treat the left wall of the left ventricle. Upon completion of treatment, electrode wires 106 can be retracted within outer shaft 102 and brush device retracted from the left ventricle.

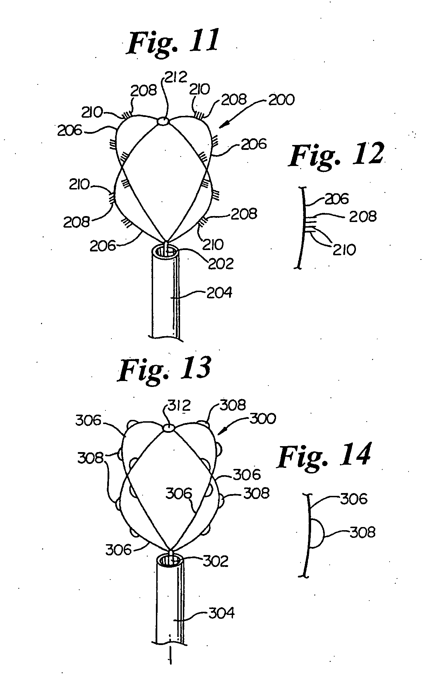

Figure 11 shows yet another embodiment of a PMR basket 200. Basket 200 is disposed at the distal end of an elongate catheter shaft 202 disposed within a guide catheter 204. Basket 200 is formed from a plurality of wires 206 each having proximal ends and distal ends. The distal ends and proximal ends, respectively are connected to each other. Wires 206 are biased to expand basket 200 transversely when unconstrained. Extending generally transversely from wires 206 are a plurality of electrode groupings 208. Each electrode grouping includes a plurality of individual elongate electrodes 210. Each of the individual electrodes 210 preferably has a diameter of about 0.001 inches to 0.009 inches. A radiopaque marker 212 can be disposed at the distal end of basket 200. Figure 12 is a fragmentary view of an electrode grouping from Figure 11.

To conduct radiofrequency energy, catheter 202 must include or be formed from a conductor, such as stainless steel or other biocompatible metal. Likewise, wires 206 and electrodes 210 must be formed from a conductor such as stainless steel, Nitinol or other biocompatible material. If wires 206 are formed from Nitinol they can be heat set to expand upon introduction of basket 200 into the left ventricle of the patient. The conductor of catheter 200 and wires 206 should be insulated to concentrate the release of RF energy at electrode groupings 208. Catheter 202 can be insulated by, for example, a surrounding layer of polyethylene, polyimide or PTFE. Wires 206 are preferably insulated by a heat shrink PTFE layer or other biocompatible insulator. Marker 212 can be formed from gold, platinum or other highly radiopaque material. Electrodes 210 can be plated with gold or other radiopaque material to enhance their visibility by fluoroscopy. Each electrode 210 can be insulated in a cylindrical ceramic housing to provide electrical insulation and thermal shielding from wires 206. Each electrode 210 is preferably between about 0.0 to 0.1 inches in length. Electrodes 210 can include a spherically shaped tip having a diameter of about 0.01 to 0.039 inches. Each grouping 208 is preferably spaced about 0.19 to 0.078 apart.

In use, guide catheter 204 is advanced to a chamber of a patient's heart, such as a left ventricle. Basket 200 is advanced in a constrained and compressed configuration to the left ventricle. Upon being advanced out of guide catheter 204 into the left ventricle, basket 204 expands. The expanded size of basket 200 should be large enough to bring electrodes 210 in contact with the wall of the left ventricle. RF energy of a sufficient magnitude is then delivered to the heart wall by way of electrodes 210. The small diameter electrodes 210 can deliver a high current density to the tissue. By placing electrodes 210 in groupings 208, the tissue can be cratered, that is a wound can be formed in the tissue which has a width greater than its depth.

Figure 13 is a perspective view yet another embodiment of a PMR basket 300. PMR basket 300 is disposed at the distal end of a catheter 302. Basket 300 is advancable to a patient's heart chamber through a guide catheter 304. Basket 300 is formed from a plurality of wires 306 biased to expand basket 300 transversely when unconstrained. Each wire 306 has a distal end and a proximal end, the proximal ends and distal ends of each wire respectively are connected to form basket 300. A plurality of loops 308 are disposed on each wire 306. The wire forming each loop has a diameter of about 0.039 inches to about 0.197 inches. Loops 308 can have a semi-circular configuration. A radiopaque marker 312 can be disposed at the distal end of basket 300. Figure 14 is a fragmentary view of basket 300 showing a side view of a loop 308 on wire 306. Each loop 308 is preferably contained within a cylindrical ceramic housing to provide electrical isolation and thermal shielding from wires 306.

The various elements of the basket embodiment of Figures 13 and 14 can be formed from the materials cited above in connection with the embodiment of Figures 11 and 12. Basket 300 is intended to be used in essentially the same manner as basket 200 to form craters in the myocardium of a patient's heart.

If can be appreciated that each of the devices disclosed herein could be made bi-polar rather than mono-polar as shown. To make these devices bi-polar, a ground electrode can be disposed proximate the electrodes shown.

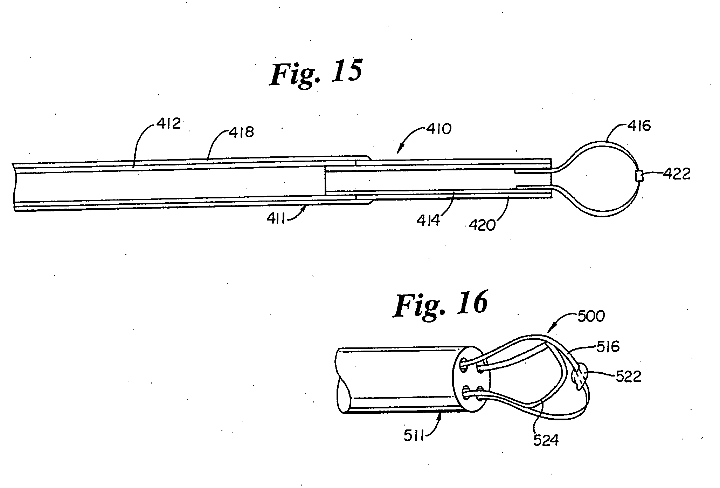

Figure 15 is a longitudinal, cross-sectional view of a catheter 410 in accordance with the present invention. Catheter 410 includes an elongate shaft 411 having a proximal portion 412 and a distal portion 414. A loop 416 is connected to catheter 410 at the distal end of shaft 411. Proximal portion 412 of shaft 411 is preferably formed from a metallic member such as a stainless steel hypotube. Portion 414 is preferably formed from a metallic member such as a Nitinol hypotube. Loop 416 is preferably formed from, for example, a Nitinol ribbon having a cross section of about 0.003 inches by about 0.005 inches as well. The connections between proximal shaft 412, distal shaft 414 and loop 416 should be formed from a solder or other conductive material such that a conductive path can be formed through shaft 411 to loop 416 for conductance of RF energy.

As one skilled in the art would recognize, an RF generator can be connected to the proximal end of shaft 411 to deliver radio frequency energy to loop 416. The strength of the RF field delivered to loop 416 should be sufficient to create the desired wound in a patient's myocardium when performing percutaneous myocardial revascularization (PMR).

To guard against injury to the vasculature through which catheter 410 is advanced, shaft 411 is insulated. Proximal portion 412 can be insulated by a layer of polyethylene 418. Distal portion 414 can be insulated by a layer of polyimide 420. These insulative materials are illustrative examples only, as other biocompatible materials may advantageously be used as insulators.

Loop 416 is preferably heat set to expand from a compressed position to be passed through a guide catheter to, for example, the left ventricle of the patient's heart. Loop 416 is preferably heat set such that as loop 416 enters the left ventricle, it will expand to a size greater than the diameter of the guide catheter lumen through which loop 416 was advanced.

A radiopaque marker 422 is preferably disposed at the distal end of loop 416. Marker 422 is preferably formed from a radiopaque material such as gold or platinum. Marker 422 should be conductively connected to loop 416 to enable marker 422 to act an electrode to deliver RF energy to a patient's myocardium. If it is desired to form craters in the patient's myocardium, the distance which marker 422 extends distally from loop 416 should be less than the maximum transverse dimension of marker 422 (a crater is a hole having a width greater than its depth).

To focus the RF energy on marker 422, loop 416 can be insulated with a material such as heat shrink PTFE. If a portion of loop 416, for example, adjacent its distal end, is left uninsulated, the uninsulated portion of loop 416 can act as an electrode. In such an instance, a very wide crater can be formed. The width of the crater being approximately equal to the transverse dimension of the uninsulated portion of loop 416. Since loop 416 can have a transverse dimension greater than that of the guide catheter lumen through which it is advanced, the crater can have a width which is in turn, greater than the diameter of the guide catheter lumen. It can be appreciated that to the extent that the transverse portion of loop 416 is insulated, it can act as a stop limiting the penetration of marker 422.

It should be noted that marker 422 and loop 416 can be pressured against the endocardium during the PMR procedure. During the PMR procedure, since the heart continues to beat, marker 422 will be motion when in contact with the heart. To absorb the movement of the heart and keep marker 422 in contact with the heart wall, it can be appreciated that loop 426 can act as a shock absorber to dampen the change in force incident to marker 422 as the heart beats.

Figure 16 is a perspective view of a distal end of an alternate embodiment 500 of a catheter in accordance with the present invention. Catheter 500 includes a shaft 511 and a loop 516 extending distally therefrom having a radiopaque marker 522 disposed on the distal end thereof. A second loop 524 extends from the distal end of shaft 511 to proximate, and proximal of the distal end of loop 516. It can be appreciated that if a portion of second loop 524 were not insulated and it were connected to ground or a lower voltage than loop 516, that it could act as a second pole to create a bi-polar RF ablation device (a second pole could be added to each of the other embodiments disclosed herein as well). A distal portion of loop 516, as well as marker 522, can be used as an electrode if left uninsulated. Insulating loop 524, loop 524 can act as a stop limiting penetration of loop 516 into the myocardium during the PMR procedure. The various components of catheter 500 can be formed from the same materials as catheter 410 and assembled in a similar manner.

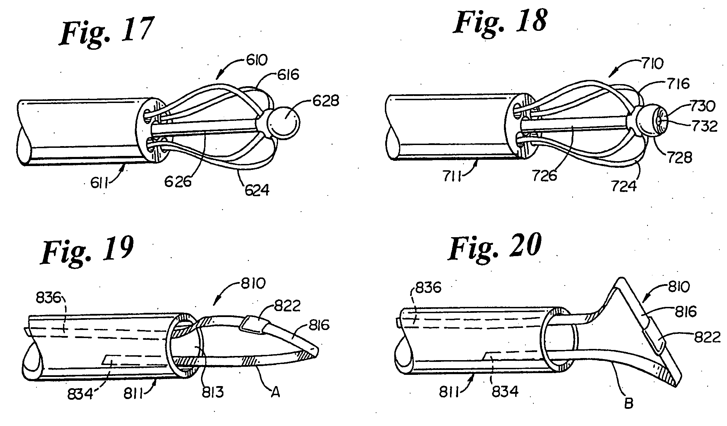

Figure 17 shows a perspective view of a distal end of yet another embodiment 610 of the catheter in accordance with the present invention. Catheter 610 includes a shaft 611 and a distal shaft extension 626. Disposed at the distal end of extension 626 is a tip 628. Shaft 626 is preferably formed from a metal such as Nitinol. Tip 628 can be a ball shaped tip formed from, for example, stainless steel. Shaft extension 626 and ball tip 628 are connected to shaft 611 by soldering or other means to form a conductive path from shaft 611 through extension 626 into ball tip 628. Ball tip 628 can then act as an electrode to form holes in the myocardium of the patient's heart during the PMR procedure.

Catheter 610 includes a first loop 616 and a second loop 624. Preferably loops 616 and 624 as well as extension 626 are insulated by a material such as heat shrink PTFE. When loops 616 and 624 are insulated, they can act as stops limiting the penetration of tip 628 into the myocardium of the patient's heart.

Figure 18 shows a perspective view of the distal end of yet another embodiment 710 of the catheter in accordance with the present invention. Catheter 710 is substantially similar to catheter 610, having a shaft 711, a conductive shaft extension 726 and electrode tip 728. Catheter 710 also includes first and second loops 716 and 724, respectively.

Each of the elements 710 are formed from the same materials in essentially the same way as that of the previous embodiments and, in particular, of catheter 610. Tip 726, however, includes a truncated surface 730. In addition, a lumen extends through the entire length of the catheter exiting at opening 732 in tip 728. During PMR, contrast media, growth factor or other drugs can be delivered to the myocardium of the patient's heart through the lumen.

Figure 19 shows a distal end of yet another embodiment 810 of the catheter in accordance with the present invention. Catheter 810 includes a shaft 811 which defines a lumen 813 extending between the proximal and distal ends of shaft 811. Loop 816 extends distally from lumen 813. Loop 816 has a first end 834 and a second end 836 which extends to the proximal end of shaft 811. End 834 is anchored to shaft 811 proximate the distal end of shaft 811. A marker 822 can be disposed on loop 816. Loop 816 is preferably formed from a metallic ribbon such as a Nitinol ribbon having cross-sectional dimensions of about 0.003 inches by about 0.005 inches. At least one end of loop 816 is connected to a radio frequency generator. Loop 816 can also be stainless steel, cold worked and heat treated into the desired geometry.

As shown in Figure 19, loop 816 is disposed in an advancement position A. In Figure 20, loop 816 is shown in a deployed position B. Loop 816 can be shifted from position A to position B by advancing end 836 distally. Loop 816 can be shifted from position B to position A by pulling end 836 proximally.

As can be seen in Figure 20, loop 816 has a substantial transversely extending distal portion. This configuration can be obtained by heat setting or pre-forming loop 816 as known to those skilled in the art. It can be appreciated that if the substantially transversely extending portion of loop 816 is left uninsulated to form an electrode, an electrode can be delivered during the PMR procedure which is substantially wider than the diameter of the guide catheter lumen through which it is advanced. Loop 816 can, however, be insulated such that only marker 822 acts as an electrode.

In use, each of the catheters of the present invention is preferably advanced percutaneously through a guide catheter extending through the aorta into the left ventricle of a patient's heart. It can be appreciated that the various embodiments can be advanced into other heart chambers as well. Once the electrode has been advanced to the patient's heart, RF energy is delivered to the electrode. The electrode is then repeatedly advanced into the patient's myocardium to create holes therein.

Numerous advantages of the invention covered by this document have been set forth in the foregoing description. It will be understood, however, that this disclosure is, in many respects, only illustrative. Changes may be made in details, particularly in matters of shape, size, and arrangement of parts without exceeding the scope of the invention. The inventions's scope is, of course, defined in the language in which the appended claims are expressed.

## Claims

1. A catheter assembly, comprising:
an elongate shaft having a proximal end and a distal end and including a conductor therein;
an insulator disposed around the conductor;
a conductive loop attached to the conductor, disposed at the distal end of the shaft, the conductive loop having a distal'end;
an electrode disposed at the distal end of the loop; and
wherein the loop can act as a shock absorber to dampen the change in force on the electrode incident to a heart beat.

2. The catheter assembly in accordance with Claim 1, wherein the elongate shaft further comprises a proximally disposed stainless steel hypotube and a distally disposed Nitinol hypotube.

3. The catheter assembly in accordance with Claim 1, wherein the elongate shaft further comprises a lumen extending between the proximal end and distal end thereof.

4. The catheter assembly in accordance with Claim 3, wherein the electrode includes a truncated tip in fluid communication with the lumen.

5. The catheter assembly in accordance with Claim 3, wherein contrast media is dispensed through the lumen and the electrode.

6. The catheter assembly in accordance with Claim 3, wherein growth factor drugs are dispensed through the lumen and the electrode.
